# EUROPEAN PATENT SPECIFICATION

(11) **EP 2 742 357 B1**
(45) Date of publication and mention of the grant of the patent: **01.02.2017**
(21) Application number: 12756392.2
(22) Date of filing: 10.08.2012
(51) Int. Cl.: G01N 33/574, G01N 33/68

(54) **HERG1 AND GLUT-1 IN COLORECTAL CANCER**
HERG1 UND GLUT-1 BEI KOLOREKTALKARZINOM
HERG1 ET GLUT-1 DANS LE CANCER COLORECTAL

(30) Priority: 12.08.2011 IT FI20110181
(43) Date of publication of application: 18.06.2014
(73) Proprietor: Universita' Degli Studi di Firenze, 50121 Firenze (IT)
(72) Inventor: ARCANGELI, Annarosa, I-50127 Firenze (IT); CROCIANI, Olivia, I-50126 Firenze (IT); LASTRAIOLI, Elena, I-50018 Scandicci (IT); ROMOLI, Raffaella, I-50133 Firenze (IT); BONI, Luca, I-16128 Genova (IT); DI COSTANZO, Francesco, I-50038 Scarperia (IT)
(74) Representative: Valenza, Silvia
(86) International application number: PCT/EP2012/065661
(87) International publication number: WO 2013/024015

(56) References cited:
- US-A1- 2005 209 280
- US-A1- 2008 206 757
- SAKASHITA ET AL: "Glut1 expression in T1 and T2 stage colorectal carcinomas: its relationship to clinicopathological features.", EUROPEAN JOURNAL OF CANCER, vol. 37, no. 2, 1 January 2001 (2001-01-01), pages 204-209, XP055013540, ISSN: 0959-8049
- DOLDERER J H ET AL: "HERG1 gene expression as a specific tumor marker in colorectal tissues", EUROPEAN JOURNAL OF SURGICAL ONCOLOGY, LONDON, GB, vol. 36, no. 1, 1 January 2010 (2010-01-01), pages 72-77, XP026825632, ISSN: 0748-7983 [retrieved on 2009-07-04]
- LASTRAIOLI E ET AL: "herg1 gene and HERG1 protein are overexpressed in colorectal cancers and regulate cell invasion of tumor cells", CANCER RESEARCH, AMERICAN ASSOCIATION FOR CANCER RESEARCH, US, vol. 64, no. 2, 15 January 2004 (2004-01-15), pages 606-611, XP002383047, ISSN: 0008-5472, DOI: 10.1158/0008-5472.CAN-03-2360 cited in the application

## Description

### Field of the invention

The present invention relates of the field of prognostic methods and particularly to a method for determining a worse prognosis in early stage non metastatic colorectal cancer patients.

### State of the art

Colorectal cancer (CRC) accounts for approximately 9.4% of total worldwide cancer causes and is the fourth most common cancer in men and the third in women. CRC survival greatly depends on the tumor stage, as TNM classification. In particular, stage II (node-negative) represents a wide spectrum of disease, where five years survival can range from 85% to 50%. Pathological tumor staging is nowadays the main predictor of prognosis and treatment in patients with CRC. However, considerable stage-independent outcome variability is observed, that likely reflects molecular heterogeneity. Accordingly, the efficacy of adjuvant chemotherapy, although well established in node-positive CRC, needs to be fully validated for TNM stage II cases. Hence more robust prognostic and predictive markers, supplementing standard clinical and pathological staging, are needed for node negative patients.

Microsatellite instability (MSI) and p53 alterations are among the most frequent tumor alterations associated with CRC oncogenesis. Indeed, a defect in mismatch repair accounts for approximately 15% colon cancers, while p53 alterations are found in half of all CRCs. Although several tumor characteristics including MSI and p53 have been proposed to influence the clinical outcome of CRC, none has been validated for clinical use yet.

Molecular candidates potentially implicated in CRC progression can be singled out from the hypoxia pathway. Tumor hypoxia is one of the key elements of tumor progression, being associated with a more aggressive phenotype and an increased propensity for metastases. Hypoxia can trigger such diverse effects since it switches on the expression of several, selected genes, through the activity of the Hypoxia Inducible transcription Factor (HIF-1). The increased expression of HIF-1-dependent proteins allows tumor cells to survive the harsh tumor microenvironment, mainly by switching on neo-angiogenesis. The impact of hypoxia on tumor progression is also mirrored by its effects on treatment: indeed hypoxia represents one of the main mediators of tumor radio- and chemo-resistance, as well as of those morphological changes, which reduce the penetration of drugs into the tumor. Endogenous markers of tumor hypoxia comprise the Vascular Endothelial Growth Factor-A (VEGFA), Carbonic anhydrase-IX (CA-IX), the Glucose Transporter 1 (Glut-1) and the Epidermal Growth Factor-Recptor (EGF-R). The expression of hypoxia markers is altered in CRC and is apparently related to CRC progression; however, their prognostic impact in CRC remains still undefined.

Voltage gated potassium channels are novel and unexpected actors in CRC establishment and progression (Arcangeli et al, Curr. Med. Chem. 2009 16: 66-93; Ousingsawat et al, Clin. Cancer Res 2007 13: 824-831). It has been provided evidence that potassium channels of the ether-a-gò-gò related gene family (hERG1) are expressed in CRC, while lacking in colonic adenomas (Lastraioli et al, Cancer Res. 2004 64: 606-611), and are functionally linked to the VEGF-A pathway (Masi et al, Br. J. Cancer. 2005 93: 781-792; Pillozzi et al, Blood 2007 110: 1238-1250). These data were confirmed by other groups both in CRC mouse models (Ousingsawat et al, Pflugers Arch-Eur.J.Physiol 2008 456:847-855) and primary human CRC (Dolderer et al, Eur J Surg Oncol 2010 36:72-77). Collectively, hERG1 expression can be considered as a reliable biomarker in CRC.

Sakashita et al (2001. Eur J Canc, 37, 204-209) discloses that grade I and II colorectal carcinomas with Glut-1 positivity have a high malignant potential. US2008206757 discloses a protocol for detection of cancer cells. The protocol comprises using cancer markers as targets for binding antibodies thereto. The list of cited markers comprises i.a. hERG1 and Glut-1.

There is a need to identify new markers to assess recurrence risk in early stage colorectal cancer patients.

### Definitions and abbreviations

CA-IX: Carbonic anhydrase-IX
CRC: colorectal cancer
EGF-R: Epidermal Growth Factor-Recptor
Glut-1: Glucose Transporter 1
HIF-1: Hypoxia Inducible transcription Factor
IHC: ImmunoHisto Chemistry
MSI: microsatellite instability
OS: overall survival
TNM:
VEGFA: Vascular Endothelial Growth Factor-A

### Summary of the invention

During a study whose objective was evaluating the prognostic impact of the hERG1 protein in conjunction with hypoxia markers (VEGF-A, GLUT-1, CA-IX and EGF-R) in a wide cohort of non metastatic CRC patients it has been surprisingly found that hERG1 positivity with Glut-1 negativity identifies a poor prognosis patients' group, within stage I-II colorectal cancer, who might benefit from adjuvant therapy, independently from the tumor stage. A classical biomolecular marker, p53, and clinico-pathological features were also included in the study. OS was taken as the main prognostic indicator.

Therefore subject-matter of the present invention is a method for determining a prognostic poor outcome in an early stage non-metastatic colorectal cancer patient, said method comprising the determination by means of IHC of hERG1 positivity along with Glut-1 negativity in a sample of the adenocarcinoma of said patient.

Further subject matter of the present invention is a kit for the prognostic method according to the invention, said kit comprising at least one container containing anti-hERG1 antibody wherein anti-hERG1 antibody recognises an extracellular epitope localized in the S5-pore region of the hERG1 protein, and at least one container containing anti-Glut-1 antibody.

### Brief description of the figures

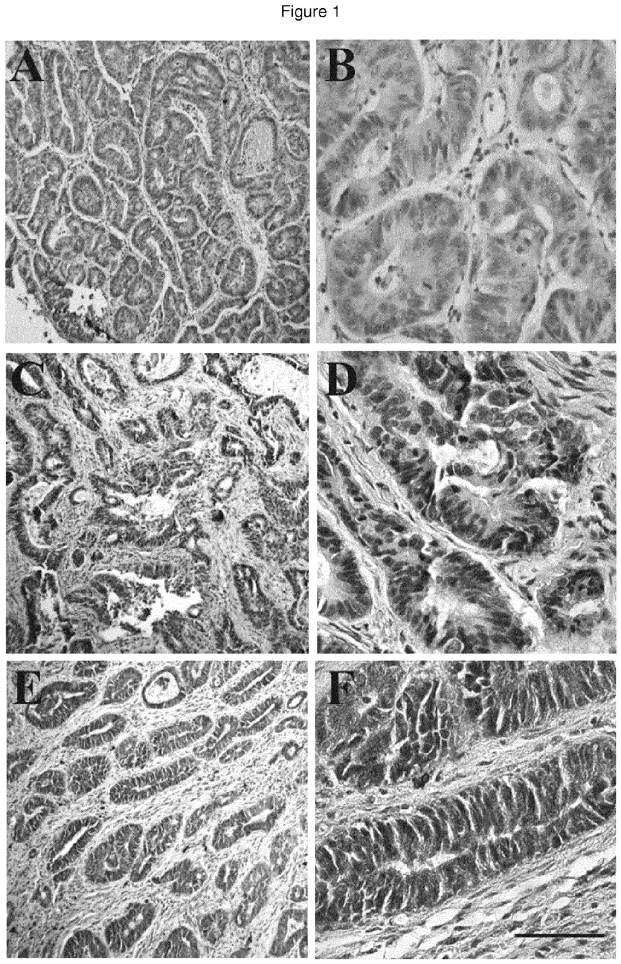

**Figure 1****. Immunohistochemistry and scoring system assessment for hERG1 in CRC specimens.**
   Figure 1 shows representative sections of a colorectal adenocarcinoma sample displaying the three different hERG1 scores are reported. **A)** Score 0 (0% of positive cells); **B)** Higher magnification of the same specimen as in A); **C)** Score 1 (1-49% of positive cells per microscopic field); **D)** Higher magnification of the same specimen as in C); **E)** Score 2 (>50% of positive cells per microscopic field); **F)** Higher magnification of the same specimen as in E). Bar: 200µm (A, C, E); 50 µm (B, D, F).

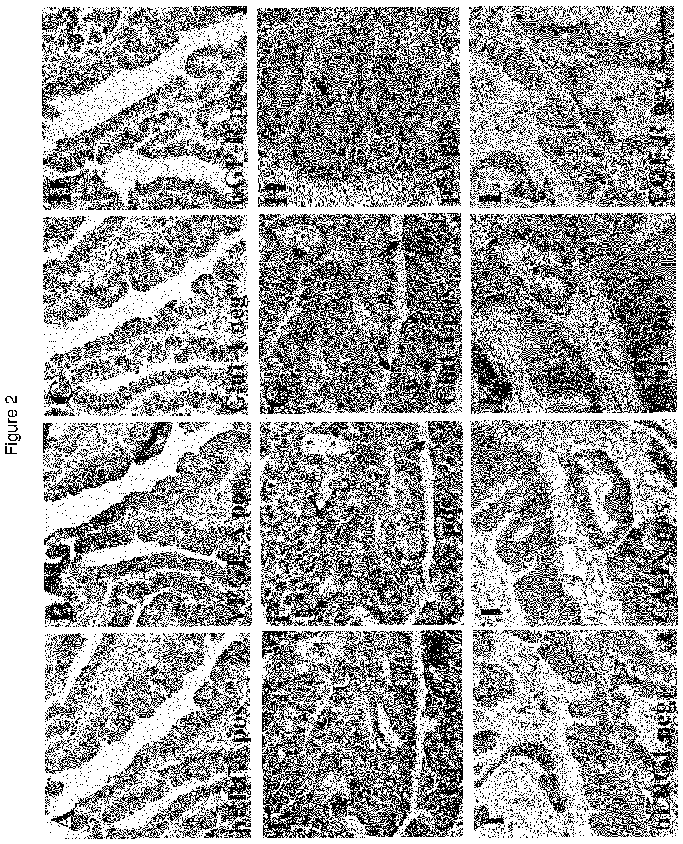

**Figure 2****. Immunohistochemical staining for hERG1, VEGF-A, Glut-1, EGF-R, CA-IX, and p53 in three different CRC specimens.**
   Figure 2 shows IHC pictures relative to three representative samples stained with the hypoxia markers (CA IX, VEGF, GLUT-1) along with hERG1**.A-D)** Immunohistochemical detection of different markers in the same TNM stage II sample: A) hERG1 pos; B) VEGF-A pos; C) Glut-1 neg; D) EGF-R pos.
   **E-H)** Immunohistochemical detection of different markers in the same TNM stage II sample: E) VEGF-A pos; F) CA-IX pos: note that CA-IX was expressed by small groups of neoplastic cells (see arrows); G) Glut-1 pos: note that, as indicated by arrows, Glut-1 expression was focal, confined to distinct areas of the tumour sample; H) p53 pos.
   **I-L)** Immunohistochemical detection of different markers in the same TNM stage I sample: I) hERG1 neg sample, in which no staining can be observed; J) CA IX pos: note that CA-IX expression was intense, although localized to small groups of cells; K) Glut-1 pos: note that expression was focal and intense; L) EGF-R neg. Bar: 50 µm.
**Figure 3** shows Kaplan-Meier curves of overall survival according to different combinations of tumor characteristics (TNM stage, Glut-1 status and hERG1 status).
**Figure 4** shows a model of the interplay between hERG1, Glut-1 and VEGF-A in CRC progression.

### Detailed description of the invention

The present invention arose from a study whose objective was to evaluate the impact of well known hypoxia markers in conjunction with a voltage dependent K+ channel, hERG1, on OS of patients with non metastatic CRC. The results of the study, although still preliminary, provide evidence that the positivity for hERG1 along with the negativity for a hypoxia marker, Glut-1, allows to identify a patients' group, within stage I and II patients, which approaches the prognosis of stage III CRC.

Hypoxia is one of the common causes of failure in cancer treatment. Indeed, hypoxia has been shown to be an important predictor of poor response to radiochemotherapy in head and neck as well as in cervix cancers. Because of this influence on therapeutic options, hypoxia represents a relevant hindrance also for treatment of CRC. However, despite its relevance, a study aimed to assess the prognostic and predictive impact of biomolecular markers belonging to the hypoxia pathway in CRC is lacking so far.

In our analysis we included the expression of hERG1 K+ channels within the classical hypoxia indicators (VEGF, CA-IX, Glut-1 and EGF-R). Indeed, voltage dependent K+ channels, including hERG1, are becoming the most original but believable biomarkers in CRC. Indeed a statistically significant association emerged between the expression of hERG1 and that of all the other hypoxia markers we analyzed. This result further confirms, in the clinical setting, what emerging from biological and experimental data, and candidates, for the first time, a potassium channel as a member of the hypoxia/angiogenesis pathway, at least in CRC.

Another pivotal hypoxia marker, VEGF-A, besides strongly associated to hERG1 expression, turned out to be moderately associated with two other markers of tumor hypoxia: CA-IX and Glut-1. Moreover, the expression of VEGF-A was strongly associated with the presence of a mutated p53, witnessed by p53 IHC positivity. This confirms that hypoxia, maybe through an increased HIF-1-mediated transcriptional activity of the *vegf-a* gene, selects p53 mutated, hypoxia-resistant clones , thus contributing to CRC progression. However, the positivity to p53 did not show any significant impact on survival of CRC patients. This is in accordance with results obtained in recent analyses.

Conversely, the univariate analysis of OS, while confirming the well proved prognostic role of the TNM stage, addresses CA-IX as an indicator of worse prognosis in CRC. The isozyme IX of Carbonic Anhydrase is involved in maintaining the extracellular pH and has been repeatedly shown to be strongly inducible by hypoxia in tumors. This is also relevant in CRC, which shows an abnormal CA-IX expression especially in the tumor areas of high proliferation. Our results further stress the relevance of CA-IX expression in CRC.

The multivariate analysis, while confirming the prognostic relevance of the TNM stage, showed that only two of the hypoxia markers we analyzed, different from CA-IX, remained in the model: hERG1, whose positivity had a negative impact on OS, and Glut-1, that, conversely, was a predictor of good prognosis, if positive. In other words, the positivity of hERG1 expression, in conjunction with the lack of Glut-1, paralleles the well established negative prognostic impact of the clinicopathological evaluation of the TNM stage.

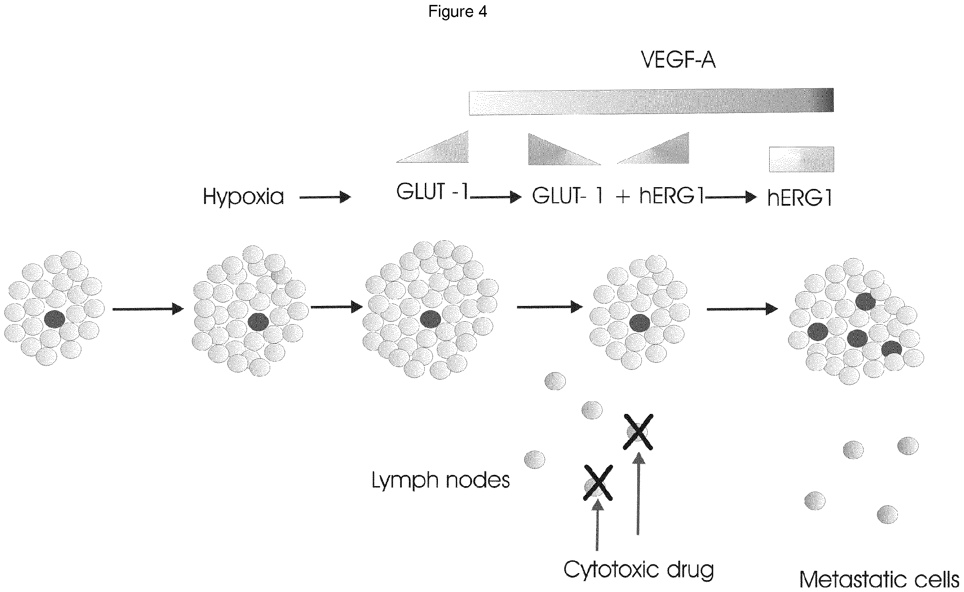

It is possible to conceive a model (Figure 4) wherein the interaction between Glut-1 and hERG1 is described and interpreted. When tumour mass reaches a critical volume a hypoxic area might be present in the central portion of the lesion. In such condition, Glut-1 expression is triggered (light to dark gray gradient triangle). Subsequently, normoxic conditions are restored due to VEGF-A-mediated angiogenesis (gray gradient rectangle) regulated by hERG1 potassium channels, whose expression increases during tumor progression (dark to light gray gradient triangle). Hence, Glut-1 expression is reduced (light to dark gray gradient triangle). In the later stages of tumour progression both VEGF-A and hERG1 are overexpressed (dark gray and white rectangles, respectively).

In such a model, Glut-1 expression is high as far as hypoxia is present within the tumor mass, while Glut-1 expression declines as soon as normoxic conditions are re-established, as a consequence of the neoangiogenesis mediated by VEGF-A, whose secretion is triggered and regulated by hERG1 channels. Hence, a condition is established, along tumor progression, which is characterized by the presence of hERG1, the continuous expression of VEGF-A and the disappearance of Glut-1. Such condition allows the establishment of a more aggressive (both drug-resistant and metastatic) phenotype.

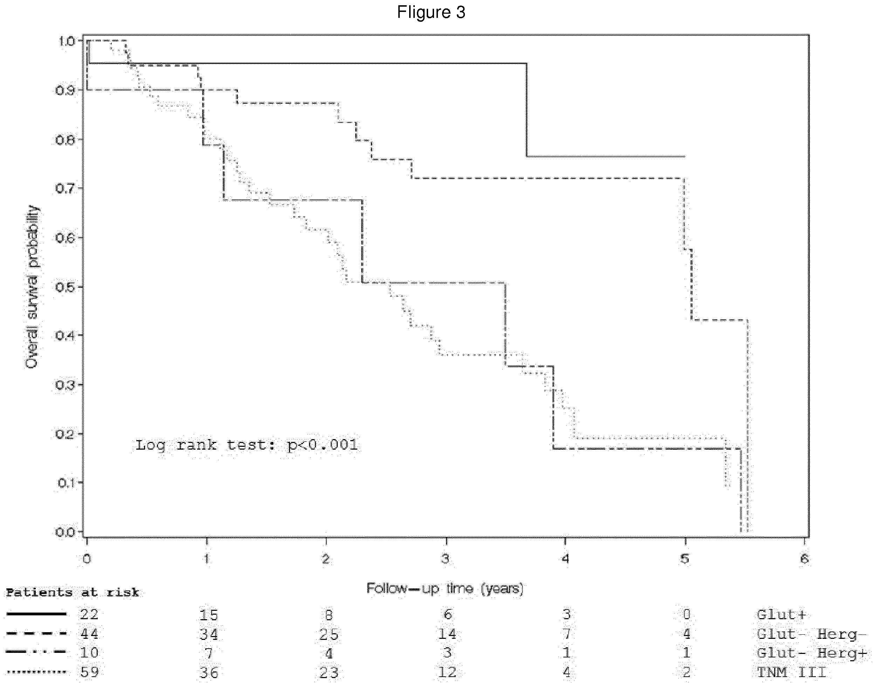

Finally, a clinical, practical, consequence comes out from such model and the results of the multivariate analysis. In fact, starting from risk scores calculated from the final multivariate model, patients were stratified into four different risk groups: A) TNM I-II, with Glut-1 pos, any hERG1; B) TNM I-II with Glut-1 neg and hERG1 neg; C) TNM I-II, with Glut-1 neg and hERG1 pos; D) TNM III, any Glut-1 and any hERG1 (see the curves in Figure 3). The three years OS probabilities inside the groups allowed to conclude that the patient group which displays positivity to hERG1 and a concomitant negativity to Glut-1, despite belonging to the TNM stages I or II, has a risk probability which fully overlaps the risk of TNM stage III patients. Although these results need to be confirmed in a wider patient sample group, we conclude that the IHC staining for hERG1 and Glut-1 might identify a subgroup of patients with regional, TNM stage I and II CRC with a worse prognosis. Actually, in current practice, the therapeutic options for CRC are directed towards stage III and, at least for those cases in which a higher recurrence risk can be identified by clinicopathological characteristics, stage II patients. Nevertheless, our data suggest that those patients with a primary tumor positive for hERG1 and negative for Glut-1 could conceivably receive an indication for a more aggressive adjuvant therapy, despite they belong to the clinicopathological stage II or even stage I.

### EXPERIMENTAL SECTION

### MATERIALS AND METHODS.

**Patients:** The study cohort includes untreated patients who underwent radical surgery with curative intent for colorectal adenocarcinomas at the Department of General Surgery and Surgical Oncology, Azienda Ospedaliero- Universitaria, Careggi, Florence.

Patients affected by viral C hepatitis or who had undergone pre-operative radiotherapy or chemotherapy for rectal cancer were excluded. Samples of tumor were collected during surgery, after obtaining an informed written consent, and immediately processed for the sample storing (see below). All the samples were classified as adenocarcinomas and staged according to the American Joint Committee on Cancer (AJCC) classification by experienced pathologists. Only samples which results to be TNM stages I to III were included in the study and further processed.

**Immunohistochemical staining:** Immunohistochemistry (IHC) was carried out on 7µm sections of colorectal adenocarcinoma samples on positively charged slides. After dewaxing and dehydrating the sections, endogenous peroxidases were blocked with a 1% H2O2 solution in PBS. Subsequently, antigen retrieval was performed as follows : a) by treatment with Proteinase K (5µg/ml) (for hERG1, VEGF-A, CA-IX and Glut-1 staining); b) by heating the samples in microwave oven at 600W in Citrate Buffer pH 6.0, for 10 (for EGF-R staining) or 20 minutes (for p53 staining). The following antibodies were used at the dilutions reported in parentheses: 1) anti-hERG1 polyclonal antibody produced in Dr Arcangeli's laboratory and distributed by Alexis Biochemicals Lausanne, Switzerland; 1:100 dilution); 2) anti-hERG1 monoclonal antibody (produced in Dr Arcangeli's laboratory and distributed by Alexis Biochemicals Lausanne, Switzerland; 1:200 dilution); 2) anti VEGF-A (Polyclonal antibody anti VEGF-A (A-20), Santa Cruz Biotechnology; Santa Cruz CA, USA, 1: 100 dilution); 3) anti-Glut-1 (Polyclonal rabbit anti-human GLUT1, Dako Cytomation; Glostrup, Denmark, 1:100 dilution); 4) anti-CA IX monoclonal antibody (Monoclonal murine antibody M75, described in Pastorekova et al, Gastroenterology 1997 112:398-408, 1:100 dilution); 5) anti-EGF-R rabbit polyclonal IgG (Santa Cruz Biotechnology; Santa Cruz CA, USA, 1: 100 dilution); 6) anti-p53 monoclonal mouse anti-human (Dako Cytomation; Glostrup, Denmark, 1:50 dilution). Incubation with the primary antibody was carried out overnight at 4°C, except for the anti-p53 antibody, that was incubated for 30 minutes at room temperature. Immunostaining was performed with a commercially available kit (PicTure Plus kit and DAB, Zymed; Carlsbad CA, USA) according to manufacturer's instructions. Samples were evaluated by two independent investigators. Staining methods were performed in accordance to the manufacturers' instructions present in the brochure included in the primary antibody's samples. For CA-IX, the method described in Pastorekova et al, Gastroenterology 1997 112:398-408 was applied.

The assignment of a positive score was performed as follows: a) for VEGF-A when the sample showed more than 10% positive cells (Galizia et al, Clin Cancer Res 2004 10: 3490-3499); b) for Glut-1 when areas displaying an unequivocal staining were detected, while areas with normal epithelium, stroma and edge effects were ignored, without using any scoring system. Red blood cells served as an internal positive control; c) for CA-IX, as reported in Korkeila et al, 2009 Br. J Cancer 100: 874-880, without using a scoring system; d) for EGF-R when at least 1% positive cells were present in the sample; e) for p53, a 10% cut-off was adopted, as reported in Veloso et al, Virchows Arch. 2000 437: 241-247.

For hERG1, besides evaluating the absence or presence of the hERG1 protein, a scoring system was used. hERG1 was expressed in the cytoplasm of neoplastic cells and at the membrane level with a diffused expression pattern. Stained sections were analyzed at a total magnification of 40x, field by field, from top left to bottom right. Each field was assigned a percentage positivity, representative of the approximate area of immunostaining. A semiquantitative scoring method that assigns IHC score as a percentage of positive tumor cells (the number of positive tumor cells over the total number of tumor cells) was used with a cutoff of 50%. Therefore, samples were considered negative (score 0) when no staining was present; a score 1 was attributed to samples displaying a positivity in a percentage ranging from 1 to 49% while a score 2 was attributed when the percentage of positive cells was more than 50%. Areas of necrosis, stroma, normal epithelium and distinct edge effects were not scored. In any case, samples were evaluated by two independent investigators.

**Statistical analysis:** The distributions of all studied patients were reported respect to their demographic, clinical and biological characteristics and were summarized as frequencies and percentage. Continuous variables were reported as median and range of variation. The following demographic and clinical variables were investigated: age at the intervention, sex, site of tumor, TNM classification and presence of colloid. The biological assessment was evaluated through the expression of: hERG1 channels, GLUT-1, VEGF-A, CA-IX, p53, and EGF-R markers. All markers were categorized as Yes/No respect to their expression. Both in the association and survival analysis, age was categorized in two groups (<70 years v ≥ 70 years). Presence of association between demographic, clinical and biological characteristics were evaluated by χ² test and Fisher's exact test, when appropriate. A two-sided P values ≤ 0.05 was considered significant. All the variables were investigated for their impact on overall survival (OS).

OS was defined as the time between intervention and death, whatever the cause. Observation time of patients alive at the last follow up was censored. Median follow up time was estimated according to Kaplan Meier inverse method (Schemper et al, Control Clin Trials 1996 17:343-346). In the univariate analysis, estimates of OS were calculated according to the Kaplan and Meier product-limit method (Kaplan et al, J. Am. Statistical Association 1958 53: 457-481). Comparisons of estimated survival curves were performed by means of the log-rank test. Hazard ratios and appropriate 95% Cls were also calculated by means of Cox's proportional hazard model. A multivariate Cox's regression model was finally fitted to evaluate the independent effect of each factor on OS. Starting from a full model including: sex, age, site of tumor, TNM, colloid, hERG1, GLUT-1, VEGF-A and CAIX non-significant variables were progressively removed according to a backward step-wise procedure, based on the likelihood ratio test. A probability of 0.10 was used both for removal and re-entry criteria. Hazard ratios and 95% confidence intervals (Cls) were reported for each variable retained in final model. Data were analyzed using the statistical software SAS 9.2 (Sas Corporation - USA).

We converted the Cox model predictor to a risk score that is directly related to an individual's probability of death from any cause. Finally, starting from the distribution of risk scores and the strata identified by the combinations of the three predictors retained in the Cox model, we empirically classified all patients into four different risk groups.

### RESULTS

### Immunohistological findings

We studied 135 colorectal cancer patients with stage I to III, collected in the period September 2001-July 2008 who underwent radical surgery for primary CRC at the General Surgery and Surgical Oncology, Azienda Ospedaliero Universitaria, Careggi, Florence. In all samples we determined the expression of hERG1 potassium channels along with several hypoxia markers (VEGF-A, CA-IX, Glut-1, EGF-R) as well as of a classical biological marker of CRC (p53) by IHC.

It has been previously reported (Lastraioli et al, Cancer Res 2004 64: 606-611) that the hERG1 protein can be detected in primary CRC samples by IHC, through a protocol employing a polyclonal antibody developed by us. In the experiments reported it was used a monoclonal antibody which recognises an extracellular epitope localized in the S5-pore region of the hERG1 protein. Statistical analysis carried out by c2 test performed on data obtained through IHC showed a statistically significant correlation (P< 0.001) between the results obtained with the two antibodies. The absence of a background signal when using the monoclonal antibody allowed us to better evaluate the percentage of positive cells per microscopic field and therefore to distinguish definite score groups.

Based on the immunoreactivity score three groups were distinguished: samples were considered negative (score 0) when no staining was present; a score 1 was attributed to samples displaying a positivity in a percentage ranging from 1 to 49% while a score 2 was attributed when the percentage of positive cells was more than 50% (see Materials and Methods). In Figure 1A-F representative sections displaying the three different hERG1 scores are reported.

Figure 2 shows IHC pictures relative to three representative samples stained with the hypoxia markers (CA IX, VEGF, GLUT-1) along with hERG1. In this case, the details of staining patterns are in the legend to Figure 2.

### Clinical characteristics

Table 1 shows the clinicopathological characteristics of the patients, as well as the distribution of the biological markers under study. Out of the 135 patients, 72 (53%) were female and 63 (47%) male. Median age was 68 years (range 40-90). Fifty-seven tumors were located in the right colon, 14 in the transverse, 33 in the left and 31 in the rectum.

**Table I: Distribution of clinicopathological and biomolecular markers and results of the univariate analysis**

| **Parameter** | **Number of Patients** | **3 year Survival** | **HR (CI 95%)** | **P-Value (LR Test)** |
|---|---|---|---|---|
| ***Age*** | | | | |
| **<70 years** | 73 (54.1 %) | 0.59 | 1 (ref.) | 0.652 |
| **≥ 70 years** | 62 (45.9 %) | 0.55 | 1.14 (0.65-1.97) | |
| ***Gender*** | | | | |
| **Female** | 72 (53.3 %) | 0.55 | 1 (ref.) | 0.588 |
| **Male** | 63 (46.7 %) | 0.59 | 1.16 (0.67-2.02) | |
| ***Tumor site*** | | | | |
| **Right Colon** | 57 (42.2 %) | 0.53 | 1 (ref.) | 0.800 |
| **Left Colon** | 33 (24.4 %) | 0.77 | 0.71 (0.34-1.51) | |
| **Transverse Colon** | 14 (104 %) | 0.52 | 0.98 (0.40-2.44) | |
| **Rectum** | 31 (23.0 %) | 0.50 | 0.80 (0.40-1.59) | |
| ***TNM Stage*** | | | | |
| **I** | 29 (21.5 %) | 0.74 | 1 (ref.) | <0.001 |
| **II** | 47 (34.8 %) | 0.74 | 0.33 (0.33-2.06) | |
| **III** | 59 (43.7 %) | 0.36 | 2.54 (1.17-5.52) | |
| ***Mucin*** | | | | |
| **No** | 100 (74.1 %) | 0.55 | 1 (ref.) | 0.504 |
| **Yes** | 35 (25.9 %) | 0.60 | 0.81 (0.44-1.49) | |
| ***hERG1*** | | | | |
| **Neg** | 104 (77.0 %) | 0.59 | 1 (ref.) | 0.203 |
| **Pos** | 31 (23.0 %) | 0.50 | 1.49 (0.80-2.76) | |
| ***TEGF-A*** | | | | |
| **Neg** | 25 (18.5 %) | 0.52 | 1 (ref.) | 0.299 |
| **Pos** | 110 (81.5 %) | 0.58 | 0.69 (0.34-1.39) | |
| ***Glut-1*** | | | | |
| **Neg** | | 0.51 | 1 (ref.) | 0.111 |
| **Pos** | 47 (34.8 %) | 0.68 | 0.60 (0.32-1.13) | |
| ***CA-IX*** | | | | |
| **Neg** | 93 (68.9 %) | 0.64 | 1 (ref.) | 0.022 |
| **Pos** | 42 (31.1 %) | 0.31 | 2.02 (1.10-3.85) | |
| ***EGF-R*** | | | | |
| **Neg** | 28 (22.2 %) | 0.55 | 1 (ref.) | 0.845 |
| **Pos** | 98 (77.8 %) | 0.58 | 0.94 (0.48-1.81) | |
| ***p53*** | | | | |
| **Neg** | 80 (61.1 %) | 0.55 | 1 (ref.) | 0.876 |
| **Pos** | 51 (38.9 %) | 0.63 | 1.05 (0.58-1.88) | |

### Relationship between biological markers and clinical characteristics

An IHC signal positive for hERG1 was observed in 23% of patients (31/135). VEGF-A expression was detected in 82 % (110/135), Glut-1 in 35 % (47/135) of the samples, while CA-IX was expressed in 42 out of 135 samples, accounting for 31 %. EGF-R and p53 were positive in 98 out of 126 (78 %) and 51 out of 131 (39 %) samples, respectively (Table 1).

It emerged a statistically significant association between hERG1 and Glut-1 (p= 0.002), hERG1 and EGF-R (p= 0.050), hERG1 and CA-IX (p=0.018), hERG1 and VEGF-A (p= 0.049), VEGF-A and p53 (p= 0.002), CA-IX and p53 (p= 0.030). A marginally statistically significant association was observed between VEGF-A and CAIX (p= 0.070), VEGF-A and Glut-1 (p= 0.080). Moreover, Glut-1 expression was significantly associated with the age of the patients (p= 0.043), while EGF-R correlated with the TNM stage (p= 0.003), as well as with the mucin content (p= 0.032). A marginally statistically significant association was also found between Glut-1 and mucin (p= 0.080), as well as between Glut-1 and the tumor site (p= 0.090).

Figure 2 shows IHC pictures relative to three representative samples stained with the hypoxia markers (CA IX, VEGF, GLUT-1) along with hERG1. In this case, the details of staining patterns are in the legend to Figure 2.

### Clinical characteristics

Table 1 shows the clinicopathological characteristics of the patients, as well as the distribution of the biological markers under study. Out of the 135 patients, 72 (53%) were female and 63 (47%) male. Median age was 68 years (range 40-90). Fifty-seven tumors were located in the right colon, 14 in the transverse, 33 in the left and 31 in the rectum.

### Relationship between biological markers and clinical characteristics

An IHC signal positive for hERG1 was observed in 23% of patients (31/135). VEGF-A expression was detected in 82 % (110/135), Glut-1 in 35 % (47/135) of the samples, while CA-IX was expressed in 42 out of 135 samples, accounting for 31 %. EGF-R and p53 were positive in 98 out of 126 (78 %) and 51 out of 131 (39 %) samples, respectively (Table 1).

It emerged a statistically significant association between hERG1 and Glut-1 (p=0.002), hERG1 and EGF-R (p=0.050), hERG1 and CA-IX (p=0.018), hERG1 and VEGF-A (p= 0.049), VEGF-A and p53 (p= 0.002), CA-IX and p53 (p= 0.030). A marginally statistically significant association was observed between VEGF-A and CAIX (p= 0.070), VEGF-A and Glut-1 (p= 0.080). Moreover, Glut-1 expression was significantly associated with the age of the patients (p= 0.043), while EGF-R correlated with the TNM stage (p= 0.003), as well as with the mucin content (p= 0.032). A marginally statistically significant association was also found between Glut-1 and mucin (p= 0.080), as well as between Glut-1 and the tumor site (p= 0.090).

### Impact on survival

Median follow up was 35 months. At the univariate analysis, whose results are reported in Table 1, the following variables turned out to have a significant impact on OS: TNM stage (stage II vs stage I: HR = 0.83, 95% CI = 0.33-2.06; stage III vs I: HR = 2.54, 95 % CI = 1.17-5.52; p<0.001) and CA-IX expression (pos vs neg : HR = 2.02, 95% CI = 1.10-3.71; p= 0.022).

Multivariate OS analysis, as reported in Table II, identified the following independent prognostic factors: TNM (stage II vs stage I: HR = 0.75, 95% CI = 0.30- 1.87; stage I vs I : HR = 3.55, 95 % CI = 1.59-7.91; p<0.001), hERG1 (pos vs neg : HR = 2.15, 95% CI = 1.12-4.13; p= 0.021) and Glut-1 (pos vs neg : HR = 0.31, 95% CI = 0.16-0.63; p= 0.001). The same results were confirmed by a sensitivity analysis performed on 122 cases without missing variables and including p53 and EGF-R information.

Starting from risk scores calculated from the final multivariate model, patients were stratified into four different risk groups: A) TNM stage I-II, with Glut-1 pos, any hERG1; B) TNM stage I-II with Glut-1 neg and hERG1 neg; C) TNM stage I-II, with Glut-1 neg and hERG1 pos; D) TNM stage III, any Glut-1 and any hERG1, with a three years OS probabilities equal to 95%, 72%, 51%,and 36%, respectively (Figure 3).

**Table II: Multivariate analysis**

| **Variable** | **Coefficient (SE)** | **Hazard Ratio** | **95% IC** | **P value** |
|---|---|---|---|---|
| ***TNM*** | | | | |
| **I** | **-** | 1 (Ref.) | | <0.001 |
| **II** | -0.23611 (0.46752) | 0.75 | 0.30-1.87 | |
| **III** | 1.26618 (0.40922) | 3.55 | 1.39-7.91 | |
| | | | | |
| ***Herg1*** | | | | |
| **Neg** | **-** | 1 (Ref.) | | |
| **Pos** | 0.76697 (0.33345) | 2.15 | 1.12-4.13 | 0.021 |
| | | | | |
| ***Glut1*** | | | | |
| **Neg** | **-** | 1 (ref.) | | |
| **Pos** | -1.15571 (0.35403) | 0.31 | 0.16-0.63 | 0.001 |

## Claims

1. A method for determining a prognostic poor outcome in an early stage non-metastatic colorectal cancer patient, said method comprising the determination by means of Immunohisto Chemistry (IHC) of hERG1 positivity along with Glut-1 negativity in a sample of the adenocarcinoma of said patient.

2. Method according to claim 1 wherein along with the determination of positivity and negativity of hERG1 and Glut-1, respectively, it is also determined by means of IHC the positivity or negativity expression of VEGF-A, CA-IX, p53, and EGF-R markers.

3. Method according to any one of claims 1-2 wherein for the determination of hERG1 positivity is employed an antibody which recognises an extracellular epitope localized in the S5-pore region of the hERG1 protein.

4. A kit for the prognostic method according to claims 1, said kit comprising at least one container containing anti-hERG1 antibody and at least one container containing anti-Glut-1 antibody; wherein anti-hERG1 antibody recognises an extracellular epitope localized in the S5-pore region of the hERG1 protein.

5. A kit according to claim 4 further comprising at least one container containing anti-VEGF-A antibody, at least one container containing anti-CA-IX antibody, at least one container containing anti-p53 antibody and at least one container containing anti-EGF-R antibody.

## Patentansprüche

1. Verfahren zum Bestimmen eines prognostisch schlechten Ausgangs bei einem Patienten mit metastasischem kolorektalem Krebs in einem frühen Stadium, wobei das Verfahren das Bestimmen von hERG1-Positivität zusammen mit Glut-1-Negativität mittels Immunohistochemie (IHC) in einer Probe von dem Adenokarzinom des Patienten umfasst.

2. Verfahren nach Anspruch 1, wobei zusammen mit der Bestimmung von Positivität und Negativität hERG1 bzw. Glut-1 ebenfalls mittels IHC die Positivität oder Negativität der Expression von VEGF-A, CA-IX, p53 und EGF-R-Markern bestimmt wird.

3. Verfahren nach einem der Ansprüche 1 bis 2, wobei für die Bestimmung von hERG1-Positivität ein Antikörper eingesetzt wird, welcher ein extrazelluläres Epitop erkennt, welches in dem S5-Porenbereich des hERG1-Proteins lokalisiert ist.

4. Kit für ein prognostisches Verfahren nach Anspruch 1, wobei das Kit wenigstens einen Behälter umfasst, der Anti-hERG1-Antikörper enthält, und wenigstens einen Behälter umfasst, welcher anti-Glut-1-Antikörper enthält, wobei der Anti-hERG1-Antikörper ein extrazelluläres Epitop erkennt, welches in dem S5-Porenbereich des hERG1-Proteins lokalisiert ist.

5. Kit nach Anspruch 4, des Weiteren umfassend wenigstens einen Behälter enthaltend Anti-VEGF-A-Antikörper, wenigstens einen Behälter enthaltend Anti-CA-IX-Antikörper, wenigstens einen Behälter enthaltend Anti-P53-Antikörper und wenigstens einen Behälter enthaltend Anti-EGF-R-Antikörper.

## Revendications

1. Procédé de détermination d'un mauvais résultat de pronostique chez un patient atteint du cancer colorectal non métastatique à un stade précoce, ledit procédé comprenant la détermination au moyen d'immunohistochimie (IHC) d'une positivité à hERG1 et d'une négativité à Glut-1 dans un échantillon de l'adénocarcinome dudit patient.

2. Procédé selon la revendication 1, dans lequel, en même temps que la détermination de la positivité et de la négativité à hERG1 et Glut-1, respectivement, l'expression de positivité ou négativité de marqueurs VEGF-A, CA-IX, p53 et EGF-R est également déterminée au moyen de l'IHC.

3. Procédé selon l'une quelconque des revendications 1 et 2, dans lequel, pour la détermination de la positivité à hERG1, est utilisé un anticorps qui reconnaît un épitope extracellulaire localisé dans la région de pores S5 de la protéine hERG1.

4. Kit pour le procédé de pronostic selon la revendication 1, ledit kit comprenant au moins un conteneur contenant un anticorps anti-hERG1 et au moins un conteneur contenant un anticorps anti-Glut-1 ; dans lequel l'anticorps anti-hERG1 reconnaît un épitope extracellulaire localisé dans la région de pores S5 de la protéine hERG1.

5. Kit selon la revendication 4, comprenant en outre au moins un conteneur contenant un anticorps anti-VEGF-A, au moins un conteneur contenant un anticorps anti CA-IX, au moins un conteneur contenant un anticorps anti-p53 et au moins un conteneur contenant un anticorps anti-EGF-R.
